(19) Europäisches Patentamt
European Patent Office
Office européen des brevets

(11) EP 2 409 677 A1

## (12) EUROPEAN PATENT APPLICATION
published in accordance with Art. 153(4) EPC

(43) Date of publication:
25.01.2012 Bulletin 2012/04

(21) Application number: 09841873.4

(22) Date of filing: 19.03.2009

(51) Int Cl.:
*A61K 6/00* (2006.01)

(86) International application number:
PCT/JP2009/055478

(87) International publication number:
WO 2010/106668 (23.09.2010 Gazette 2010/38)

(84) Designated Contracting States:
AT BE BG CH CY CZ DE DK EE ES FI FR GB GR HR HU IE IS IT LI LT LU LV MC MK MT NL NO PL PT RO SE SI SK TR

(71) Applicants:
• Southcoast Dental Co., Ltd.
Osaka 530-0015 (JP)
• Sofsera Corporation
Tokyo 160-0022 (JP)

(72) Inventors:
• TAMAKI, Kozo
Osaka-city
Osaka 530-0015 (JP)

• KAWABE, Karl Kazushige
Tokyo 160-0022 (JP)
• KOGAI, Yasumichi
Tokyo 160-0022 (JP)

(74) Representative: Zinkler, Franz
Patentanwälte Schoppe, Zimmermann, Stöckeler Zinkler & Partner
Postfach 246
82043 Pullach (DE)

## (54) TOOTH SURFACE REPAIRING MATERIAL

(57) Provided is a tooth surface repairing material which can produce an effect in a short time while preventing fine particles from shedding. The tooth surface repairing material contains fine calcium phosphate particles, and is **characterized in that** the fine calcium phosphate particles are highly crystalline sintered calcium phosphate and have an average particle diameter in the range of 20-100 nm.

[FIG.2]

EP 2 409 677 A1

## Description

### Technical Field

The present invention relates to a tooth surface repairing material which coats the tooth surface and repairs the scratches on the tooth surface and more specifically, relates to the tooth surface repairing material containing calcium phosphate.

### Background Technology

Conventionally, it is known for an apatite combined in a composition for a dental health has protein adsorption ability and it is not only effective in controlling bacterial plaque, but also contributes to advance recalcification of an enamel surface (Non-Patent Document 1). Next, for example, Patent Document 1, it is suggested a substance, wherein a spherical apatite is combined as a dentifrice composition which can be easily inserted in gaps between the teeth or fissures with high mobility. In addition, Patent Document 2, it is suggested also a dentifrice composition to repair an irregularity of the tooth surface by directly forming a coated layer of the apatite on the tooth surface.

Recently, fine material can be used, i.e. a large number of 'Nano sized' powders have been reported. According to patent document 3, it has been reported that the fine scratches or early decalcifying portions of the tooth surface can be recalcified by combining the apatite with the powder diameter more than 0.05 $\mu$m and less than 1.0 $\mu$m and calcium phosphate even in the field of the composition for the dental health.

### Prior Art Documents

Patent Document 1: JP H04-247020, A
Patent document 2: JP H06-24929, A
Patent document 3: JP H09-202717, A
Non-Patent Document 1: Journal of Dental Health Vol 38, 510-511(1988)

### Disclosure of the Invention

### Problem to be Solved by the Invention

However, in an actual living environment, it is considered the people are struggling for wearing out of the tooth surface or filling of the tooth surface due to nano sized fine particles or formation of the apatite membrane due to expected recalcification without avoiding wearing out of the tooth due to dentifrice derived abrasives; more specifically, shedding of the fine particles due to an impact of saliva or food or drinks. Furthermore, an acute effect cannot be expected since comparatively more time is required to form the apatite membrane due to recalcification. Therefore, the object of the present invention is to provide a tooth surface repairing material which can produce an effect in a short time while preventing fine particles from shedding. Means for Solving the Problem

The present invention was completed with information that the sorbability, stability of the tooth surface, more specifically anti-solubility after adsorption has been improved as compared to the conventional composition with an amorphous apatite by mixing highly crystalline sintered calcium phosphate into the tooth surface repairing material to control shading due to scratches and tooth surface as a result of committed investigations carried out to achieve the object. Moreover, the composition is effective in the tooth surface repairing material since it not only recalcifies the fine scratches, but coating or filing is carried out by using the relevant fine particles by mixing fine highly crystalline calcium phosphate with minute size.

The present invention (1) is a tooth surface repairing material containing fine calcium phosphate particles, wherein the fine calcium phosphate particles are highly crystalline calcium phosphate and have an average particle diameter in the range of 20 to 100 nm.

The present invention (2) is the tooth surface repairing material according to invention (1), wherein the fine calcium phosphate particles are manufactured by a method which includes a mixing process to mix primary particles containing calcium phosphate and a fusion inhibitor and a sintering process to convert the primary particles included in the particle mixture into highly crystalline calcium phosphate particles by exposing the particle mixture obtained by the mixing process.

The present invention (3) is the tooth surface repairing material according to invention (2), wherein the fusion inhibitor used in the mixing process contains calcium ions.

The present invention (4) is the tooth surface repairing material according to any of inventions (1) to (3), wherein an abundance ratio (Ca/P) of the calcium atom with respect to the phosphorus atom on the surface of the fine calcium phosphate particles is 1.6 or more.

The present invention (5) is the tooth surface repairing material according to any one of inventions (1) to (4), wherein at least some of the fine calcium phosphate particles are in the form of a particle.

The present invention (6) is the tooth surface repairing material according to any one of inventions (1) to (5), wherein at least some of the fine calcium phosphate particles are fluroapatite.

The present invention (7) is the tooth surface repairing material according to any one of inventions (1) to (6), wherein at least some of the fine calcium phosphate particles have a coefficient of variation of 20% or less.

Effect of the Invention

The present invention (1) can produce an effect, wherein the fine calcium phosphate particles gets tightly adhered to the surface of the tooth and smoothly covers the surface to noticeably improve sorbability and stability; more specifically, the anti-solubility to the surface of the tooth by having an average particle diameter of the highly crystalline fine calcium phosphate particles. In addition, it also produces an effect wherein the crystallinity of the fine calcium phosphate particles gets increased due to sintering and it becomes hard to dissolve in water and fuse with the other particles.

The present invention (2) can produce an effect wherein the calcium phosphate is sintered under the existence of the fusion inhibitor and therefore, it remains in the primary particles state and the particle diameter become smaller which in turns gets easily adhered to the surface of the tooth and gets filled more quickly and easily.

The present invention (3) can produce an effect with high increase in the sorbability to the surface of the tooth.

The present invention (4) can produce an effect wherein adsorption for the tooth surface with a minus charge becomes easy due to increase in the abundance ratio of the calcium atoms carrying the plus charge on the surface of the fine calcium phosphate particles.

The present invention (5) can produce an effect wherein the fine calcium phosphate can uniformly get adhered without any gaps on the surface of the tooth since it contains particles in the form of a particle.

The present invention (6) can produce an effect wherein the surface of the tooth is not only filled by the fine calcium phosphate particles but at the same time it can be coated by fluorine.

The present invention (7) can produce an effect wherein the particles get uniformly adhered to the surface of the tooth since the scattering of the particles is extremely less.

**Brief Description of the Drawings**

Fig. 1 is a scanning electron micrograph of the tooth enamel surface.

Fig. 2 is a scanning electron micrograph of the tooth enamel surface after having applied a particle of Manufacturing Example 1.

Fig. 3 is a scanning electron micrograph of the tooth enamel surface after having applied a particle of Manufacturing Example 2.

Fig. 4 is a scanning electron micrograph of the tooth enamel surface after having applied a particle of Manufacturing Example 3.

Fig. 5 is a scanning electron micrograph of the tooth enamel surface after having applied a particle of comparison Manufacturing Example 1.

Fig. 6 is a scanning electron micrograph of the tooth enamel surface after a particle of Manufacturing Example 2 was applied and allowed to stand in water overnight.

Fig. 7 is a scanning electron micrograph of the tooth enamel surface after a particle of Manufacturing Example 3 was applied and allowed to stand in water overnight.

Fig. 8 is a scanning electron micrograph of the tooth enamel surface after a particle of Comparative Example 1 was applied and allowed to stand in water overnight.

Fig. 9 is a scanning electron micrograph of the initial dental work part of the tooth enamel surface after having applied a particle of Manufacturing Example 2.

Fig. 10 is a scanning electron micrograph of the crack part of the tooth enamel surface after having applied a particle of Manufacturing Example 2.

Fig. 11 is a scanning electron micrograph of the crack part of the tooth enamel surface after having applied a particle of Manufacturing Example 3.

Fig. 12 is a scanning electron micrograph of the dentine surface.

Fig. 13 is a scanning electron micrograph of the dentine surface after having applied a particle of Manufacturing Example 1.

Fig. 14 is a scanning electron micrograph of the dentine surface after having applied a particle of Manufacturing Example 2.

Fig. 15 is a scanning electron micrograph of the dentine surface after having applied a particle of Manufacturing Example 3.

Fig. 16 is a scanning electron micrograph of the dentine surface after having applied a particle of comparison Manufacturing Example 1.

**Best Mode for Carrying Our the Invention**

The tooth surface repairing material according to the preferred example contains highly crystalline sintered fine calcium phosphate particles. Besides, it may contain various types of abrasives, wetting agents, detergents, thickeners, preservatives, sweeteners, perfume material and water and various types of other medical constituents as optional constituents. In this, it is particularly suitable to add wetting agents from the point of view of flocculation of the particles.

The crystalline calcium phosphate according to the preferred example has an average particle diameter in the range of 20 to 100 nm (more preferably 20 to 90 nm and furthermore, preferably 20 to 50 nm). For the tooth surface repairing material according to the present example, the average particle diameter is taken in the range described above and fine structure on the surface of the tooth is covered and filled with the fine highly crystalline calcium phosphate particles and hollow portions formed on the surface of the tooth are repaired. In addition, as a result of repairing the tooth surface, the effect of preventing or treating sensory sensitivity can be produced as well. The tooth surface repairing material can produce the effect in a short time as compared to the recalcination of the calcium phosphate since the fine calcium phosphate particles directly cover the surface of the tooth and fill the scratches. Note that, if the average particle diameter is less than 20 nm, the particles get washed away with blood flow and do not work sufficiently as the tooth surface repairing material. If the average particle diameter is more than 100 nm, the particles easily flocculate and the fine particles cannot easily adhere uniformly on the surface of the tooth. On the other hand, if the particle diameter is less than 100 nm, the particles cannot easily overlap and the surface of the tooth gets closely filled and therefore, it produces the effect of increasing whitening and glazing of the tooth. Furthermore, if the particle diameter is less than 100 nm, it gets adhered to so-called "carious tooth" (preferentially in the case of a large amount of the tooth surface repairing material) on the surface of the tooth. Note that, if the highly crystalline fine calcium phosphate particles are sintered by using the mixing process described later, the particle diameter may be in the range of 20 to 300 nm. However, if the particle diameter is more than 300 nm, the fine particle itself becomes large and it cannot easily adhere to the surface of the tooth. Therefore, it does not fully work as the tooth surface repairing material. The cause of the phenomenon is not known, but it is considered to be because the weight of the particle increases with increase in the size of the particle and therefore, though it is adhered to the surface of the tooth, it instantly comes off. Note that, it is better to calculate the average particle diameter and coefficient of variation by measuring the particle diameter of at least 100 or more primary particles. The coefficient of variation is preferably to be 20% or less, more preferably to be 18% or less and furthermore, preferably to be 15% or less. Note that, the 'coefficient of variation' is a value indicating the variation in the particle diameter between the particles which can be calculated by using the formula: standard deviation, average particle diameter Í100 (%). The highly crystalline calcium phosphate has no specific shape but it is better to have a shape so the filling can be done in fine structure of the tooth surface. For example, it may have a particle shape or rod shape. However, the particle shape fine calcium particles are preferable from the point of view of close filling to the tooth surface. Note that, when it has a rod shape, the average particle diameter and coefficient of variation is determined on the basis of the longitudinal length.

$$\text{coefficient of variation} = (\text{standard deviation}) / (\text{average particle diameter}) \times 100$$

Here, as for the calcium phosphate, for example, hydroxyapatite ($Ca_{10}(PO_4)_6(OH)_2$), fluoroapatite ($Ca_{10}(PO_4)_6F_2$), and $Ca_{10}(PO_4)_6CL_2$, etc. are given. In addition, the crystalline calcium phosphate may contain a compound, wherein some of the calcium ion and/or hydroxylion and/or phosphoric ion are replaced with strontium ion, barium ion, sodium ion, bicarbonate, carbonate ion, fluoride ion and chloride ion, etc. or calcium phosphate ($Ca_3(PO_4)_2$), calcium metaphosphate ($Ca(PO_3)_2$) and octacalcium phosphate (OCP). Among the above examples, hydroxylapatite and/or fluroapatie are preferably used.

Next, $Ca_{10}(PO_4)_6(OH)_2$ is preferable to be the present on the surface of the calcium phosphate particles (particles of the calcium phosphate) according to the preferred example. $Ca_{10}(PO_4)_6(OH)_2$ is preferable to be the present on the surface of the calcium phosphate and it is preferably to be 0.1 % weight for the total quantity of the calcium phosphate, but more preferably to be 50% weight or more. In addition, the calcium phosphate may contain tricalcium phosphate produced at the time of sintering the non-crystalline hydroxyapatite. The calcium phosphate according to the present example has an excellent stability in the living environment and has an affinity to the living composition and therefore, it is suitable to be used as the material for medical treatment; more specifically, material for the dental surgery. In addition, the calcium phosphate according to the present invention is hard to dissolve in the living body. Therefore, it can maintain bioactivity for a long period of time in the living body.

Next, as for the highly crystalline calcium phosphate, a sintered compact (also called as calcium phosphate ceramics) of the highly crystalline calcium phosphate wherein calcium phosphate is sintered (burned) is used. The sintered compact of the highly crystalline calcium phosphate can be obtained by sintering the non-crystalline calcium phosphate. Specifically, for example, the sintered compact of the highly crystalline calcium phosphate can be obtained by sintering using the method described later. The crystallinity can be increased by sintering the calcium phosphate. For example, the solubility when introduced in the living body (tooth surface fine structure) can be reduced. In addition, if highly crystalline sintered calcium phosphate is used, sorbability and stability to the tooth surface and anti-solubility after adsorption is improved. The level of the crystallinity of the calcium phosphate can be measured by using the X ray diffraction method (XRD). Specifically, the smaller the half width of the peak showing the surface of each crystal, the larger the crystallinity is. Here, the 'high crystallinity' of the highly crystalline calcium phosphate of the present invention signifies when d = 2.814, the half width is 0.7 or less (preferably 0.5 or less).

As for the other optional constituents, no specific abrasives, but for example, calcium carbonate, calcium pyrophosphate, silicic acid anhydride, aluminum silicate, aluminium hydroxide and calcium hydrogen phosphate, etc. are given. No specific wetting agents, but for example, glycerin, sorbitol, propylene glycol, polyethylene glycol, maltitol, xylitol, lactitol, erythritol and trehalose are given. Among these wetting agents, more specifically, propylene glycol is preferably used. As for the surfactants, alkyl sulphate, alkyl benzene sulfonate, sucrose esters of fatty acids and sodium lauryl sulphate, etc. are given. No specific thickeners, but for example, hydroxyethyl cellulose, carrageenan, carboxy ethyl cellulose, carboxy vinyl polymer, sodium polyacrylate, xanthan gum, carboxymethylcellulose sodium, cellulose gum, sodium alginate, hydroxypropylcellulose, Cyamoposis Gum, Chondroitin sulfate sodium salt, polyacrylic acid and polymethacrylic acid, etc. are given. No specific preservatives, but for example, sodium benzoate, methylparaben, p-hydroxybenzoate ester and Alkyldiaminoethylglycine hydrochloride, etc. are given. No specific sweeteners, but for example, saccharin sodium, xylitol and stevia extract, etc. are given. No specific perfume materials, but for example, menthol, orange oil, spearmint oil, peppermint oil, lemon oil, eucalyptus oil and methyl salicylate, etc. are given. No specific medicinal components, but agents making nervous sensations poor such as potassium nitrate and fluorine coating agents such as sodium monofluorophosphate, etc. are given.

The tooth surface repairing material according to the preferred example is prepared in dosage forms such as gel form and paste form. Any constituents described above can be included in any form. In addition, the above constituents can be added as the gelatinizing agent in the case of the gel form composition and the thickener in the case of the paste from composition. More specifically, if it becomes high salt concentrated due to a buffering solution system, the non-ionic polymer i.e. hydroxyethyl cellulose, Cyamoposis Gum, hydroxypropylcellulose and tragacanth gum, etc. can be included.

In the tooth surface repairing material according to the preferred example, the content of the fine calcium phosphate particles is preferable to be 0.1 to 70% weight, more preferably to be 1 to 50% weight and furthermore, preferably to be 5 to 30% weight with respect to the total amount. The content of the abrasive is preferably to be 0.1 to 50% weight, more preferably to be 0.2 to 30% weight and more preferably to be 1 to 20% weight with respect to the total amount of the tooth surface repairing material. The content of the wetting agents is preferably to be 0.1 to 70% weight, more preferably to be 0.5 to 50% weight and more preferably to be 1 to 40% weight with respect to the total amount of the tooth surface repairing material. The content of the surfactant is preferably to be 0.01 to 10% weight, more preferably to be 0.02 to 5% weight and more preferably to be 0.05 to 10% weight with respect to the total amount of the tooth surface repairing material. The content of the thickener is preferably to be 0.01 to 20% weight, more preferably to be 0.02 to 15% weight and more preferably to be 0.05 to 10% weight with respect to the total amount of the tooth surface repairing material. The content of the preservative is preferably to be 0.01 to 10% weight, more preferably to be 0.02 to 5% weight and more preferably to be 0.05 to 1% weight with respect to the total amount of the tooth surface repairing material. The content of the sweetener is preferably to be 0.01 to 5% weight, more preferably to be 0.02 to 3% weight and more preferably to be 0.05 to 1% weight with respect to the total amount of the tooth surface repairing material. The content of the perfume material is preferably to be 0.01 to 5% weight, more preferably to be 0.02 to 3% weight and more preferably to be 0.05 to 1% weight with respect to the total amount of the tooth surface repairing material. The contents of the medical constituents can be set according to the constituents, but for example, it is preferably to be 0.01 to 20% weight, more preferably 0.02 to 10% weight and furthermore, preferably to be 0.05 to 5% weight with respect to the total amount of the tooth surface repairing material.

((Method for Manufacturing))

Next, the method for manufacturing the crystalline calcium phosphate according to the preferred example is described. The crystalline calcium phosphate can be obtained by sintering the amorphous calcium phosphate. The calcium phosphate may be manufactured artificially by using the known manufacturing methods such as a wet process, drying process, hydrolysis and hydrothermal technique as well. In addition, it may be a naturally derived substance obtained from the bone and tooth, etc. In addition, the lower limit of the sintering temperature is preferably to be 500°C or more. If the sintering temperature is less than 500°C, sintering may not be sufficient. On the other hand, the upper limit of the sintering temperature is preferably to be1800°C or less, more preferably to be 1250°C or less and furthermore, preferably to be 1200°C or less. If the sintering temperature is more than 1800°C, the calcium phosphate may get decomposed. Accordingly, the calcium phosphate is hard to dissolve in the human body (highly crystalline) and can be manufactured by setting the sintering temperature within the range described above. In addition, the sintering time can be set accordingly and it has no specific limit. Note that, the particle may get fused due to sintering, but in this case, the particles after sintering can be used after milling.

The highly crystalline fine calcium phosphate particles according to the preferred example are more specifically preferred to be manufactured by using the methods described later. The method for manufacturing highly crystalline fine calcium phosphate particles according to the preferred example is preferred to be a method which at least includes the distributed calcination (sintering) from the mixing process and sintering process. The fine particles obtained by using the distributed calcination method reflects the particle diameter of the primary particles as it is and therefore, it can be easily adjusted to the average particle diameter in the specified range. In addition, the method for manufacturing according to the preferred example may include the primary particle generation process and removal process. These processes, for example, can be performed in the sequence as a primary particle generation process, mixing process, sintering process and removal process.

(Primary particle generation process)

The primary particle generation process has no specification limitation as long as it is the process which can generate the fine calcium phosphate particles. It may be used upon proper selection according to the raw material of the highly crystalline calcium phosphate particles to be manufactured. For example, the particles of the calcium phosphate (CaP) get precipitated when the phosphoric acid is dropped into the calcium hydroxide slurry under the normal temperature.

Similar to the fine calcium phosphate particles according to the preferred example, the method of generating a group of very fine (nano meter sized) primary particles with even particle diameter (particle distribution is limited) has no specific limitations, but for example, the method according to the Japanese published unexamined application No. 2002-137910 can be used. In other words, the fine calcium phosphate (hydroxyapatite) particles (primary particles) can be systhesized by solubilizing and mixing the calcium solution and phosphoric acid solution in an emulsion phase of detergent/water/ oil system and allows it to react above the clouding point of the detergent. In addition, at that time, the size of the fine calcium phosphate particles can be controlled by changing the functional group and the percentage of the hydrophilicity/hydrophobicity ratio of the detergent.

The following describes principles of producing the hydroxyapatite fine particles. In the foregoing method in which a solution of calcium and a solution of phosphoric acid are dissolved and mixed in an emulsion phase of a detergent/water/oil system to produce fine particles of hydroxyapatite, hydroxyapatite cores grow in the micelles of the detergent to form crystals. With the reaction temperature at or above the cloud point of the detergent, the thermodynamic stability of the micelles can be controlled. That is, by increasing the reaction temperature at or above the cloud point of the detergent, the force acting upon the detergent to form micelles can be weakened. As one can imagine, this will increase the driving force that promotes crystal growth of hydroxyapatite in the micelles but that has been restricted by the force maintaining the micelles. As a result, the force maintaining the micelles and preventing crystal growth can be overcome. The shape of crystals can be controlled by taking advantage of this mechanism.

Important factors involving formation of micelles by the detergent are the functional groups (hydrophilic moieties) of the detergent, and a ratio of hydrophilic group to hydrophobic group within the molecule. These factors determine stability of the micelles and the cloud point. Different types of detergents have different cloud points. Thus, by suitably selecting a detergent, it is possible to change the functional groups, and the ratio of hydrophilic group to hydrophobic group. As a result, the size of hydroxyapatite fine particles can be controlled.

Note that, there is no specific type of detergent to be used in the method described above and other known types disclosed in the Japanese published unexamined application No. 5-1711 such as anion, cation, zwitter ion, non-ionic detergent can be selected and used. Among the aforementioned detergents, when the detergent is a non-ionic detergent, the crystal shape wherein the mechanism described above is used is easy to control as it has the clouding point of the detergent. More specifically, as for non-ionic detergent, polyoxyethylene alkyl ether, polyoxyethylene allyl ether, polyoxyethylene alkyl allyl ether, polyoxyethylene derivatives, oxyethylene oxypropylene block copolymer, sorbitan fatty acid ester, polyoxyethylene sorbitan fatty acid ester, glycerine fatty acid ester, polyoxyethylene fatty acid ester and polyoxyethylene alkylamine, etc. can be used. In addition, as for the cation detergent, a quaternary ammonium base such as stearylamine hydrochloride, Lauryltrimethylammonium Chloride, alkyl benzene dimenthyl ammonium Chloride, etc. can be used. As for an anion detergent, a higher alcohol sulphuric ester base such as sodium lauryl alcohol sulphate ester and sodium oleyl alcohol sulphate ester, etc., alkylsulfate base such as sodium lauryl sulphate and ammonium lauryl sulphate and alkyl allyl sulphonic acid base such as sodium dodecylbenzenesulfonate and sodium dodecylnaphthalenesulphonate, etc. can be used. As for an amphoteric detergent, alkyl betaine group, alkyl amidobetaine group and amine oxide group can be used. The above detergents can be used in combination of one or more than two types. In this, more specifically, Pentaethylene glycol dodecyl ether is preferably to be used from the point of view of clouding point and solubility.

In addition, as an oil phase, which can be used in the above method, for example, hydrocarbon base such as toluene, xylene, hexane, dodecane and cyclohexane, etc., halogenated hydrocarbon base such as chlorobenzene and chloroform, etc., ether base such as diethyl ether, etc., alcohol base such as butanol, etc., ketone base such as methyl isobutyl ketone, cyclohexanone, etc. are given. One type or two types of these solvents can be selected according to the detergent to be used so one of the detergents described above is soluble in the solvent with less water solubility. Among these, more specifically dodecane is preferably to be used from the point of view of water solubility and detergent solubility. Besides this, the reaction temperature, reaction time and additive amount of the raw material is desired to be accepted after selecting optimum conditions according to the composition of the primary particles. However, the upper limit of the reaction temperature is preferably to be the temperature at which the solvent does not get boiled as it is the reaction of an aqueous solution and more preferably to be 90°C or less.

In addition, the present process may include the process of cleaning the generated primary particles with water and the process of recovering the primary particles by centrifugation and filtering.

(Mixing Process)

The mixing process is the process to mix the primary particles and the fusion inhibitor. The fusion inhibitor is inserted between the particles in the primary particle groups obtained by using the primary particle generation process beforehand so the fusion of the primary particles in the subsequent sintering process can be prevented. Note that, the mixture of the primary particles and the fusion inhibitor obtained by using the mixing process is called 'Particle mixture.'

There is no specific'fusion inhibitor' as long as it can prevent the fusion between the primary particles, but it is desired to be non-volatile in the sintering temperature of the subsequent sintering process. This is because the fusion of the primary particles can be definitely prevented as they do not disappear from the primary space between the primary particles during the sintering process as they are non-volatile under the sintering temperature conditions. However, the particles are not required to have a 100% non-volatility in the sintering temperature. The non-volatility between the primary particles is desired to be 10% or more after completing the sintering process. In addition, the fusion inhibitor may be the inhibitor which gets chemically decomposed due to the heat after completing the sintering process. In other words, if it remains after completing the sintering process, it is not necessary to be the same substance (compound) before and after starting the sintering process.

In addition, the fusion inhibitor is preferably to be the substance which is soluble in the solvent, more specifically an aquatic solvent. As described above, when the substance soluble in the solvent is used as the fusion inhibitor, it (for example, calcium carbonate) can be removed just by suspending the fine calcium phosphate particles with the fusion inhibitor mixed in it into the aquatic solvent such as purified water. More specifically, when the fusion inhibitor soluble in the aquatic solvent is used, the organic solvent is not required to be used while removing the fusion inhibitor and therefore, equipment for using the organic solvent and organic solvent waste disposal is not required in the removal process. Due to this, it is said the fusion inhibitor can be more easily removed from the fine calcium phosphate particles. There is no specific solvent, but for example, as for the aquatic solvent, water, ethanol and methanol, etc. are given and as for organic solvents, acetone and toluene, etc. are given.

In addition, the aquatic solvent may contain chelate compounds such as oxalate, ethylene diamine, bipyridine and ethylenediamine tetraacetate, etc. to increase the solubility of the fusion inhibitor into the water. Furthermore, the aquatic solvent may contain electrolytic ions such as sodium chloride, ammonium nitrate and potassium carbonate, etc. to increase the solubility of the fusion inhibitor into the water.

Here, the solubility of the detergent soluble with respect to the solvent is said to be preferable since the removal efficiency increases with the increase in the solubility. The solubility is preferably to be 0.01 g or more, more preferably 1 g or more and most preferably 10 g or more when the amount of the dissolved substance with respect to the 100 g solvent is considered to be the solubility.

As for the specific fusion inhibitor, the calcium base (or complex) such as calcium chloride, calcium oxide, calcium sulfate, calcium nitrate, calcium carbonate, calcium hydroxide, calcium acetate and calcium citrate, etc., potassium salt such as potassium chloride, potassium oxide, potassium sulfate, potassium nitrate, potassium carbonate, potassium hydroxide and potassium phosphate, etc. and the sodium group such as natrium chloride, sodium oxide, sodium sulfate, sodium nitrate, sodium carbonate, sodium hydroxide and sodium phosphate, etc. are given.

Note that, there is no specific method for mixing the primary particles and the fusion inhibitor according to the mixing process. It may be the method of mixing the solid primary particles and solid fusion inhibitor by using a blender or it may be the method of dispersing the primary particles in the solution of the fusion inhibitor. However, two solid substances are difficult to mix uniformly and therefore, the later method is said to be preferably used to insert the fusion inhibitor uniformly and properly between the primary particles. If the later method is used, the fusion inhibitor solution, wherein primary particles are dispersed is desired to be made dry. The state wherein the primary particles and the fusion inhibitor are mixed uniformly can be kept for a long period of time. According to the examples described later, the particle mixture is obtained by dispersing the primary particles of the hydroxyapatite (HAp) 0.5 g in the saturated aqueous solution of the calcium carbonate and drying at 80°C.

In addition, the mixing process may be the process wherein the solution containing the high polymer compound having one of the groups such as carboxyl group, sulfate group, sulfonic acid group, phosphate group, phosphonate group and amino group or their bases in the side chain and primary particles are mixed and metallic salt (alkali metallic salt and/or alkali earth metallic salt and/or transition metallic salt) is further added. If the above method is used, the contact between the calcium phosphate (hydroxyapatite (HAp)) can be certainly inhibited in the mixing process of the fusion inhibitor since the high polymer compound gets adhered to the surface of the calcium phosphate or hydroxyapatite (HAp). After that, the fusion inhibitor can be certainly precipitated on the surface of the calcium phosphate or hydroxyapatite (HAp). Note that, in the following description, the high polymer compound having one of the groups such as carboxyl group, sulfate group, sulfonic acid group, phosphate group, phosphonate group and amino group or their bases in the side chain is simply called a 'high polymer compound.'

The high polymer compound is not restricted to be a compound having one of the groups such as carboxyl group, sulfate group, sulfonic acid group, phosphate group, phosphonate group and amino group or their bases in the side chain. For example, for the high polymer compound having a carboxyl group in the side chain, polyacrylic acid, polymethacrylic acid, sodium polyacrylate, sodium polymethacrylate, carboxymethyl cellulose, styrene unhydrous maleate copolymer, etc. are given. For the high polymer compound having a sulfate group in the side chain, polyacrylic alkyl sulfate ester, polymethacrylic alkyl sulfate ester and polystyrene sulfate, etc. are given. For the high polymer compound having a sulfonic acid group in the side chain, polyacrylic alkyl sulphonic ester, polymethacrylic alkyl sulphonic ester and polystyrene sulfonic acid, etc. are given. For the high polymer compound having a phosphate group in the side chain, polyacrylic alkyl phosphate ester, polymethacrylic alkyl phosphate ester, polystyrene phosphate and polyacryloyl amino methyl phosphonate, etc. are given. For the high polymer compound having a phosphonate group in the side chain, polyacrylic alkyl phosphonate ester, polymethacrylic alkyl phosphonate ester, polystyrene phosphonate and polyacryloyl amino methyl phosphonate, etc. are given. For the high polymer compound having an amino group in the side chain, polyacrylamide, polyvinylamine, polymethacrylic amino alkyl ester, polyaminostyrene, polypeptide and protein, etc. are given. Note that, one of the high polymer compounds or the mixture of many types of high polymer compounds can be used in the mixing process.

Note that, the high polymer compound has no specific molecular weight, but preferably more than 100 g/mol and less than 1,000,000 g/mol, more preferably more than 500 g/mol and less than 500,000 g/mol and more preferably more than 1,000 g/mol and less than 300,000 g/mol can be used. If the molecular weight of the high polymer compound is less than the desired range, the percentage of getting into the space between the primary particles gets decreased and the percentage of prohibiting the contact between the primary particles gets decreased. In addition, if the molecular weight of the high polymer compound exceeds the desired range, the operation performance such as decreasing the solubility of the high polymer compound and increasing the consistency of the solution containing the high polymer compound becomes poor and therefore, not preferred.

Note that, the solution containing the high polymer compound is desired to be an aqueous solution. This is because the sintered particles of the calcium phosphate (hydroxyapatite (HAp)) get dissolved under strong acidic conditions. Note that, the pH of the aqueous solution containing the high polymer compound is more than 5 and less than 14. The aqueous solution containing the high polymer compound has no specific pH if HAp particles are insoluble. The aqueous solution containing the high polymer compound is desired to be an aqueous solution such as an ammonia aqueous solution, sodium hydroxide and potassium hydroxide with pH adjusted, wherein a high polymer compound is dissolved in the distilled water, ion exchanged water.

In addition, the concentration of the high polymer compound included in the aqueous solution is preferably more than 0.001% W/v and less than 50% W/v, more preferably more than 0.005% W/v and less than 30% W/v and furthermore, preferably more than 0.01% W/v and less than 10% W/v. If the concentration of the high polymer compound is less than the desired range, the amount of getting into the space between the primary particles gets decreased and the percentage of prohibiting the contact between the primary particles gets decreased. If the concentration of the high polymer compound exceeds the desired range, the operation performance such as difficulty in dissolving the high polymer compound and increasing the viscosity of the solution containing the high polymer compound becomes poor and therefore, not preferred.

In the mixing process of the present invention, the solution containing the high polymer compound and the primary particles are mixed. It is better to add the primary particles in the aqueous solution and agitated so the primary particles get dispersed into the solution. In the method for manufacturing the highly crystalline calcium phosphate according to the present invention, it is possible to add one of the groups of carboxyl group, sulfate group, sulfonic acid group, phosphate group, phosphonate group and amino group or their bases to the surface of the primary particle after adhering the high polymer compound to the surface of the primary particles by using the above operation. At that time, a carboxyl group, sulfate group, sulfonic acid group, phosphate group, phosphonate group and amino group are the present in the ionic state in the solution.

Then, if the metallic salt (alkali metallic salt and/or alkali earth metallic salt and/or transition metallic salt) is further added to the solution wherein the solution containing the high polymer compound and the primary particles are mixed, the carboxylate ion, sulfate ion, sulphonic acid ion, phosphate ion, phosphonate ion and amino ion the present on the surface of the primary particles and the metallic ion (alkali metallic salt and/or alkali earth metallic salt and/or transition metallic salt) gets bonded and carboxylate, sulfate, sulphonate, phosphate, phosphonate and amino acid salt gets generated on the surface of the primary particles. The carboxylate, sulfate, sulphonate, phosphate, phosphonate and amino acid salt of the metallic salt (alkali metallic salt and/or alkali earth metallic salt and/or transition metallic salt) functions as the fusion inhibitor. Therefore, the primary particles on the surface of which the carboxylate, sulfate, sulphonate, phosphate, phosphonate and amino acid salt of the metallic salt (alkali metallic salt and/or alkali earth metallic salt and/or transition metallic salt) are generated is supposedly the 'Particle mixture.' Note that, the carboxylate, sulfate, sulphonate, phosphate, phosphonate and amino acid salt of the metallic salt (alkali metallic salt and/or alkali earth metallic salt and/or transition metallic salt) gets precipitated and it is better to collect the precipitate and provide to the sintering process described later after drying it. For the drying, the method of heating (preferably more than 0°C and less than 200°C, more preferably more than 20°C and less than 150°C and furthermore, preferably more than 40°C and less than 120°C) under a reduced pressure condition (preferably more than $1 \times 10^5$ Pa and less than $1 \times 10^{-5}$ Pa, more preferably more than $1 \times 10^3$ Pa and less than $1 \times 10^{-3}$ Pa and furthermore, preferably more than $1 \times 10^2$ Pa and less than $1 \times 10^{-2}$ Pa) is given. Note that, the heating under the reduced pressure condition is desired in drying since the drying temperature can be reduced, but it can be performed under atmospheric pressure as well.

No specific alkyl metallic base but, for example, natrium chloride, sodium hypochlorite, sodium chlorite, sodium bromide, sodium iodide, sodium iodate, sodium oxide, sodium peroxide, sodium sulfate, sodium thiosulfate, sodium selenate, sodium nitrite, sodium nitrate, sodium fluoride, sodium carbonate, sodium hydroxide, potassium chloride, potassium hypochlorite, potassium chlorite, potassium bromide, potassium iodide, potassium iodate, potassium oxide, potassium peroxide, potassium sulfate, potassium thiosulfate, potassium selenate, potassium nitrite, potassium nitrate, potassium fluoride, potassium carbonate, potassium hydroxide etc. can be used.

In addition, as for alkali earth metallic base, for example, magnesium chloride, magnesium hypochlorite, magnesium chlorite, magnesium bromide, magnesium iodide, magnesium iodate, magnesium oxide, magnesium peroxide, magnesium sulfate, magnesium thiosulfate, magnesium selenate, magnesium nitrite, magnesium nitrate, magnesium fluoride, magnesium carbonate, magnesium hydroxide, calcium chloride, calcium hypochlorite, calcium chlorite, calcium bromide, pota calcium ssium iodide, calcium iodate, calcium oxide, calcium peroxide, calcium sulfate, calcium thiosulfate, calcium selenate, calcium nitrite, calcium nitrate, calcium fluoride, calcium carbonate, calcium hydroxide etc. can be used.

In addition, zinc chloride, zinc hypochlorite, zinc chlorite, zinc bromide, zinc iodide, zinc iodate, zinc oxide, zinc peroxide, zinc sulfate, zinc thiosulfate, zinc selenate, zinc nitrite, zinc nitrate, zinc fluoride, zinc carbonate, zinc hydroxide, iron chloride, iron hypochlorite, iron chlorite, iron bromide, iron iodide, calcium iodate, iron oxide, iron peroxide, iron sulfate, iron thiosulfate, iron selenate, iron nitrite, iron nitrate, iron fluoride, iron carbonate, iron hydroxide etc. can be used. In addition, nickel compounds can be used as well.

Note that, the metallic salt (alkali metallic salt, alkali earth metallic salt and transition metallic salt) added in the solution, wherein the solution containing the high polymer compound and the primary particles are mixed may be the mixture of 1 type or more than two types. In addition, metallic salt (alkali metallic salt, alkali earth metallic salt and transition metallic salt) can be used in the solid state but, it is preferably to be used in the form of aqueous solution to enable to add it uniformly and control the concentration to be added. In addition, the amount (concentration) of the metallic salt (alkali metallic salt and/or alkali earth metallic salt and/or transition metallic salt) to be added is not specific as long as carboxylate, sulfate, sulphonate, phosphate, phosphonate and amino acid salt of the metal (alkali metal and/or alkali earth metal and/or transition metal) gets generated after bonding with the carboxylate ion, sulfate ion, sulphonate ion, phosphate ion, phosphonate ion and amino ion present on the surface of the primary particles. The quantity may be decided upon proper investigations.

Note that, the carboxylate, sulfate, sulphonate, phosphate, phosphonate and amino acid salt of the metallic salt (alkali metallic salt and/or alkali earth metallic salt and/or transition metallic salt) generated on the surface of the primary particles gets thermally decomposed in the sintering process described later and becomes oxides of the metal (alkali metal and/or alkali earth metal and/or transition metal). For example, if the calcium polyacrylate gets generated on the surface of the primary particles, it becomes calcium oxide due to the sintering process. Note that, since the metallic compounds (alkali metallic compounds and/or alkali earth metallic compounds (for example, calcium oxide) and/or transition metallic compounds) are soluble in water, they can be easily removed by using the removal process described later.

Note that, the sodium polyacrylate is soluble in water and thus can be used as it is, as the fusion inhibitor in the mixing process but, the calcium polyacrylate is insoluble in water and thus it is desired to be precipitated on the surface of the primary particles by adding the calcium salts once only the polyacrylic acid gets adhered to the surface of the primary particles. In addition, the high polymer compounds get decomposed at the time of calcination of the primary particles at the high temperature (about 300°C or more). Therefore, the metallic salts of the high polymer compounds are desired to be precipitated on the surface of the primary particles to make them function as the fusion inhibitor after calcination. However, if the primary particles are calcinated (heat treatment) under the temperature at which the high polymer compounds do not decompose (do not become soft), the metallic salts of the high polymer compounds are not required to be precipitated on the surface of the primary particles.

The method for manufacturing the fine calcium phosphate particles according to the preferred example was described above but the fusion inhibitor to be used in the mixing process is desired to be containing the calcium ion. In other words, as the fusion inhibitor, previously described calcium salt or high polymer compounds and calcium salt are desired to be used. Due to this, an outflow of the calcium atoms from the fine calcium phosphate can be suppressed in the sintering process and therefore, the composition ratio (Ca/P value) of the calcium atoms with respect to the phosphorus atoms present on the surface of the fine calcium phosphate particles gets increased. Due to this, the fine calcium phosphate particles can be easily adhered to the tooth surface. The mechanism is not clear but the adhering strength of the fine particles to the tooth surface is considered to be high, since the tooth surface has minus charge and large amount of calcium with plus charge with respect to minus charge is present on the surface of the particle. Note that, the abundance ratio (Ca/P) of the calcium atoms with respect to the phosphate atom present on the surface of the fine calcium phosphate particles according to the preferred example is desired to be 1.60 or more. The abundance ratio of the atom is measured by using XPS.

(Sintering process)

The sintering process is the process to convert the primary particles included in the particle mixture to the highly crystalline fine calcium phosphate particles (sintered compact particles) by exposing the particle mixture obtained in the mixing process at the sintering temperature. Since the fusion inhibitor is inserted between the particles of the primary particles, though the particles are exposed under the high temperature in the sintering process, the fusion of the particles can be prevented.

The sintering temperature in the sintering process is desired to be set properly so that the hardness of the highly crystalline fine calcium phosphate particles gets the desired hardness. For example, preferably 100°C to 1800°C , more preferably 150°C to 1500°C and most preferably 200°C to 1200°C . Note that, the sintering time should be set properly on the basis of the hardness of the desired highly crystalline fine calcium phosphate particles. In the example described later, sintering is carried out for 1 hour at 800°C.

Note that, there is no specific apparatuse for sintering process but, calcination furnace available in the market may be selected and used according to the manufacturing scale and manufacturing conditions.

(Removal process)

The removal process is the process to remove the fusion inhibitor present between the particles of the highly crystalline fine calcium phosphate particles obtained by the sintering process.

The procedure and the method of removal may be selected according to the fusion inhibitor used in the mixing process. For example, if the fusion inhibitor soluble in the solvent is used, only the fusion inhibitor can be dissolved and removed by using the solvent in which highly crystalline fine calcium phosphate particles are insoluble (insoluble) and the solvent in which the fusion inhibitor is soluble (soluble). There is no specific solvent to be used as long as it is the solvent which meets the conditions described above. It may be aquatic solvent or organic solvent. For example, as for the aquatic solvent, water, ethanol and methanol etc. are given and as for organic solvent, acetone and toluene etc. are given.

In addition, the aquatic solvent may contain chelate compounds such as oxalate, ethylene diamine, bipyridine and ethylenediamine tetraacetate etc. to increase the solubility of the fusion inhibitor into water. Furthermore, the aquatic solvent may contain electrolytic ions such as sodium chloride, ammonium nitrate and potassium carbonate etc. to increase the solubility of the fusion inhibitor into water.

However, the solvent to be used in the removal process is desired to be aquatic solvent due to the reasons such as no need of appratuse for using organic solvent, no need of organic solvent waste process, high stability of manufacturing operation and low risk to the environment.

Note that, the removal process is desired to be carried out with pH 4.0 to pH 12.0 since the highly crystalline sintered calcium phosphate particles or hydroxyapatite (HAp) gets dissolved when pH is less than 4.0.

However, when the fusion inhibitor is to be removed by using the solvent, only the highly crystalline calcium phosphate particles may be collected by filtering and centrifugation after suspending the highly crystalline calcium phosphate containing the fusion inhibitor obtained by the sintering process into the solvent. The above operation may be performed once or twice in the method of manufacturing of highly crystalline calcium phosphate according to the preferred example. The removal rate of the fusion inhibitor of highly crystalline calcium phosphate is said to be further improved when the above operation is performed several times. However, the above operation is not to be performed unnecessarily due to the reasons such as complex manufacturing process, high manufacturing cost and low recovery rate of the highly crystalline calcium phosphate. Accordingly, the frequency of the above operation is to be decided properly on the basis of the removal rate of the target fusion inhibitor.

Note that, the present process may also contain the process to classify the particle diameter more uniformly.

Other than the method of removing the fusion inhibitor by using the solvent, it can also be removed by using the magnetic substance by using a magnet in the fusion inhibitor. More specifically, highly crystalline calcium phosphate particle group (coarse highly crystalline calcium phosphate particle) containing the fusion inhibitor obtained by using the sintering process is suspended into the proper solvent (like water) and then the suspended solution is magnetized. Only the fusion inhibitor gets adhered to the magnets and highly crystalline calcium phosphate particles which do not get adhered to the magnet are collected. In addition, the method wherein coarse highly crystalline calcium phosphate particles are pulverized and converted to the pulverulent body without suspending into the solvent and then the fusion inhibitor is separated by using the magnet can be used. However, when the particles are suspended in the solution, highly crystalline calcium phosphate particles and the fusion inhibitor can be removed easily and the removal rate of the fusion inhibitor becomes high. Note that, highly crystalline calcium phosphate particles to which this method can be applied, is desired to be non magnetic body or weak magnetic body.

((Properties))

In the case of the highly crystalline calcium phosphate particles manufactured by the method of manufacturing of these particles according to the preferred example, the primary particles are prevented from fusing due to the action of the fusion inhibitor and therefore, majority of the particles keep the primary particle state. Accordingly, the majority of the highly crystalline calcium phosphate particles can be broken into the primary particles made of single crystal or particle group (primary particles made of single crystal), wherein the primary particles made of the single crystal are grouped by performing ionic interaction at the time of suspending the highly crystalline calcium phosphate particles into the solvent.

The highly crystalline calcium phosphate particles according to the preferred example has large surface area, since majority of the particles are the primary particles made of single crystal or particle group (primary particles made of single crystal), wherein the primary particles made of the single crystal are grouped by performing ionic interaction with good dispersibility into the solvent and secondary particles are not formed.

In the method of evaluating whether the highly crystalline fine calcium phosphate particles exist in the form of the primary particle or not, the particle diameter is measured by using the electron microscopy and the particle diameter is measured when they are suspended into the solvent by using dynamic light scattering technique and when both the measurement results are almost equal it can be determined that most of the highly crystalline fine calcium phosphate particles are in the form of primary particle. Further, when the measurement result obtained by dynamic light scattering technique is greater than the measurement result obtained by electron microscopy, it can be determined that the secondary particles has formed due to the fusion of the primary particles.

Note that, there is no specific solver for dispersing the highly crystalline fine calcium phosphate particles according to the preferred example as long as the particles are insoluble therein. For example, water or alcohol base such as methanol and ethanol, ketone base such as acetone, methyl ethyl ketone, methyl isobutyl ketone, cyclohexane, amide base such as N,N-dimethylformamide, sulfoxide base such as dimethyl sulfoxide, hydro carbon base such as xylene, hexane, dodecane, cyclohexanone, halogenated hydrocarbon base such as chlorobenzene, chloform and ether base such as diethyl ether, dioxane are given. For these solvents, one or more that two types can be selected according to the intended use.

The percentage of the primary particles made of single crystal and particle group (primary particles made of single crystal), wherein the primary particles made of the single crystal are grouped by performing ionic interaction can be calculated by finding the percentage of the particles having the particle diameter almost same as that of the particle diameter of the primary particles found by using the electronic microscope.

Note that, it may change according to the calcium phosphate raw material, type of the fusion inhibitor and sintering conditions but, according to the method for manufacturing highly crystalline fine calcium phosphate particles pertaining to the preferred example, the primary particles made of single crystals may be at least 50% or more, more preferably 60% or more and further more preferably 70% or more.

The tooth surface repairing material according to the preferred example can be obtained by mixing highly crystalline fine calcium phosphate particles obtained by the method of manufacturing and gelatinizing agent, and binder.

(Amount to be used)

The tooth surface repairing material according to the preferred example can be used as quasi drug or cosmetics such as dentifrice, liquid dentifrice, and medical equipment such as dental surface abrasive. The tooth surface repairing material according to the preferred example not only fills the hollow portions of the tooth surface but also can be used for beatification of tooth such as increasing the glossiness of the tooth as it gets smoothly adhered to the surface of the enamel. Furthermore, the tooth surface repairing material according to the preferred example can also be used as a therapeutic agent for preventing sensory sensitivity since it gets smoothly adhered to the dentine surface as well and form a dentine coating by forming an adsorption layer. Here, 'hyperesthesia' means temporary acute pain in the tooth at the time of eating hot or cold eatables. The hyperesthesia may cause due to thinning of gums due to periodontitis, exposure of dentine at the portion of dental root and loss of enamel after formation of the tooth. More specifically, the symptoms of feeling sharp pain in tooth due to the stimuli such as cold water or brushing when dental tubules of the dentine get exposed due to cuneiform defect, enamel abrasion and gingival recession is called as hypersensitive dentine. Normally, the tooth consists of enamel, dentine and cementum. The enamel is exposed on the surface and covers the dentine. Note that, the tooth repairing material according to the preferred example produce an effect when fine calcium phosphate particles, 1 mg or more are applied once or more than one time in a day on the surface of the tooth as the dentifrice.

Examples

Manufacturing Example 1 (Manufacturing of calcium phosphate fine particles) (Primary particle generation process)

Dodecane $[CH_3(CH_2)_{10}CH_3]$ was used as continuous oil phase and pentaethylene glycol dodecyl ether $[CH_3(CH_2)_{10}CH_2O(CH_2O)_4CH_2CH_2OH]$ with clouding point 31°C was used as non-ionic detergent. The continuous oil phase, 40ml containing non-ionic detergent, 0.5g was prepared at the room temperature. Next, calcium hydroxide $[Ca(OH)_2]$ dispersed aqueous solution 10ml was added into the continuous oil phase prepared in the ratio of 2.5mol/l and water-in-oil solution (W/O solvent) was prepared. While stirring W/O solvent, sodium dihydrogen phosphate $[(KH_2PO_4)]$ solution, 10ml was added into it in the ratio of 1.5mol/l. Then, it is allowed to react at the room temperature for 24 hours while stirring. Next, the reactant obtained was cleaned by centrifugation and the primary particle group of hydroxylapatite (HAp) was obtained.

(Mixing Process)

An aqueous solution, 100ml with pH 12.0 containing sodium polyacrylate, 1.0g (weight-average molecular weight 15,000g/mol manufactured by ALDRICH) was taken and the primary particle group of hydroxylapatite (HAp), 1.0g was dispersed into it. The sodium polyacrylate was allowed to adhere to the surface of the same particles. The pH of the aqueous solution was measured by using pH meter D-24SE manufactured by Horiba Ltd.

Next, the aqueous solution of calcium nitrate $[Ca_3(NO)_2]$, 100ml was added to the dispersion liquid prepared above in the ratio of 0.12mol/l and sodium polyacrylate was made to precipitated on the surface of the same primary particles. The sodium polyacrylate is the fusion inhibitor. The precipitate obtained as a result was collected and dried under the reduced pressure (about 0.1Pa) at 80°C and particle mixture was obtained.

(Sintering Process)

The particle mixture was put into the crucible and sintered for one hour at the sintering temperature at 800°C. At that time, the calcium polyacrylate was thermally decomposed which turns into the calcium oxide [CaO]. The residual ratio of the sintered calcium oxide was 25% or more.

(Removal Process)

A 50mmol /1 ammonium nitrate $[NH_4NO_3]$ water solution was prepared to increase the solubility of the water of the fusion inhibitor. Next, a sintering body provided with the above process was suspended in the 500ml water solution prepared above, separated and washed using centrifugal separation, and suspended in distilled water. Then, the fusion inhibitor and ammonium nitrate were removed by separating and washing with centrifugal separation in the same manner, and the high crystalline hydroxyapatite (HAp) fine particles were collected. Detailed information of hydroxyapatite fine particles provided by these processes is complied below.

Half bandwidth of RD: 0.2 (d = 2.814)
Shape: Spherical
Average particle size (using electron microscope): 28nm
Variation index: 14%

Manufacturing Example 2

The operation similar to the manufacturing example 1 except the difference in the reaction temperature which was taken as 30°C in the process of generation of the primary particles was performed and fine calcium phosphate particles were obtained in the manufacturing example 2. The detailed information about the fine hydroxyapatite particles is described below.

Half bandwidth of RD: 0.2 (d = 2.814)
Shape: Spherical
Average particle size (using electron microscope): 43nm
Variation index: 12%

Manufacturing Example 3

The operation similar to the manufacturing example 1 except the difference in the reaction temperature which was taken as 80°C in the process of generation of the primary particles was performed and fine hydroxyapatite particles were obtained in the manufacturing example 3.

Half bandwidth of RD:0. 2 (d=2. 814)
Shape: Rod-shaped
Average particle size (using electron microscope): (Long axis direction: Length) 167nm (Short axis direction: Thickness) 52nm
Variation index: (Long axis direction) 28% (Short axis direction) 30%

Manufacturing Example 4

The operation similar to the manufacturing example 2 except the difference of omitting the addition of calcium nitrate was performed by using $Ca(OH)_2$ as the fusion inhibitor and hydroxyapatite particles were obtained in the manufacturing example 4.

Half bandwidth of RD: 0.2 (d = 2.814)
Shape: Spherical
Average particle size (using electron microscope): 57nm
Variation index: 43%

Comparison Manufacturing Example 1

Under similar conditions as that of the manufacturing example 2, only the primary particle generation process was performed and without performing the subsequent process such as mixing process and sintering process, unbaked fine hydroxyapatite particles were obtained in the comparison manufacturing example 1. The detailed information about the fine hydroxyapatite particles is described below.

Half bandwidth of RD: 0.8 (d = 2.814)

Shape: Particle-shaped
Average particle size (using electron microscope): 42nm
Variation index: 17%

Examples 1-5 (Tooth Surface Repairing Material)

The tooth surface repairing material of the present invention is prepared by the method wherein the highly crystalline fine hydroxyapatite particles are mixed with the other constituents according to the information and considered it as dosage forms such as quasi drug or cosmetics such as dentifrice, liquid dentifrice, and medical equipment such as dental surface abrasive. The representative composition is enumerated below. However the described substances are an example, and the constitution substance and the combination rate are not specifically limited thereto, and should be appropriately set.

Example 1 Toothpaste agent composition

Hydroxyapatite 30.0%
Glycerin 36.0%
Cellulose gum 1.0%
Refined water 33.0%

Example 2 Toothpaste agent composition

Hydroxyapatite 50.0%
Glycerin 25.0%
Polyethylene glycol 4.0%
Cellulose gum 1.0%
Refined water 20.0%

Example 3 Toothpaste agent composition

Hydroxyapatite 30.0%
Aluminium hydroxide 5.0%
Potassium nitrate 5.0%
Glycerin 25.0%
Propylene glycol 10.0%
Refined water 25.0%

Example 4 Toothpaste agent composition

Calcium phosphate 35.0%
Hydroxyapatite 10.0%
Glycerin 30.0%
Monofluorophosphate 0.5%
Sodium lauryl sulfate 0.5%
Refined water 24.0%

Example 5 Toothpaste agent composition

Hydroxyapatite 30.0%
Aluminium hydroxide 10.0%
Silicic acid anhydride 5.0%
Glycerin 25.0%
Propylene glycol 10.0%
Refined water 20.0%

(Particle surface composition testing)

Ca/p measurement result was shown for the fine hydroxyapatite particles obtained in the manufacturing example 2 and 4 by using ICP and XPS. In addition, the data regarding unbaked fine hydroxyapatite particles before the mixing process and sintering process obtained in the manufacturing example 2 and fine hydroxyapatite particles sintered by conventional method was obtained. Here, the conventional method means the method of sintering without performing the mixing process. In ICP, since the particle is measured by dissolving it, the average composition ratio on the surface and the internal part of the particle can be obtained. On the other hand, the average composition ratio on the surface of the particle can be obtained in XPS since only the surface of the particle is measured. Below, the table 1 shows the ICP result and table 2 shows the XPS result. Note that, in ICP, the test material was dissolved in the hydrochloric acid and respective analytical curve was created on the basis of calcium ion standard solution and phosphate ion standard solution. Then the respective amount of ion included in the measured sample was determined. In XPS, the sample container made of aluminum was filled with the test sample and an element ratio of Ca and P was measured under the following conditions after sufficiently reducing the pressure.

Device to be used: 1600S type X-rays photoelectron spectrum device made in PHI company Measurement condition: X-ray source MgK$\alpha$ (400W)
Analysis region: 0.8x2.0mm

[Table 1]

| Table 1: Ca/P measurement results before and after sintering with ICP | |
| --- | --- |
| | Ca/P (Atom ratio) |
| Unbaked | 1.69 |
| Conventional Method | 1.67 |
| Ca(OH)$_2$ (Manufacturing Example 4) | 1.72 |
| PAA-Ca (Manufacturing Example 2) | 1.72 |

[Table 2]

| Table 2: Ca/P measurement results before and after sintering with XPS | |
| --- | --- |
| | Ca/P (Atom ratio) |
| Unbaked | 1.59 |
| Conventional Method | 1.51 |
| Ca(OH)$_2$ (Manufacturing Example 4) | 1.64 |
| PAA-Ca (Manufacturing Example 2) | 1.62 |

From these results, it was understood that the calcium on unbaked apatite surface as well as the apatite surface obtained by using the conventional method was less than the theoretical value. Note that, since the hydroxyapatite has an excellent ion exchanging ability, the particles not necessarily turns into the theoretical value. More specifically, defluxion of the calcium ion is remarkable on the surface of the fine hydroxyapatite particles. According to Table 2, the defluxion of the calcium ion is remarkable on the surface in the conventional sintering whereas, it is controlled on the surface of

the fine hydroxyapatite particles obtained in the manufacturing example 2 and 4. Accordingly, it was understood that since the percentage of the calcium ion is seen to be more on the surface, when compared with the sintered hydroxyapatite according to the conventional method, large amount of the particles with positive charge has been obtained. From the subsequent adhesion test as well, it is understood that removal is hard in the particles obtained in the manufacturing example 2 than unbaked particles.

(Coat application test on enamel)

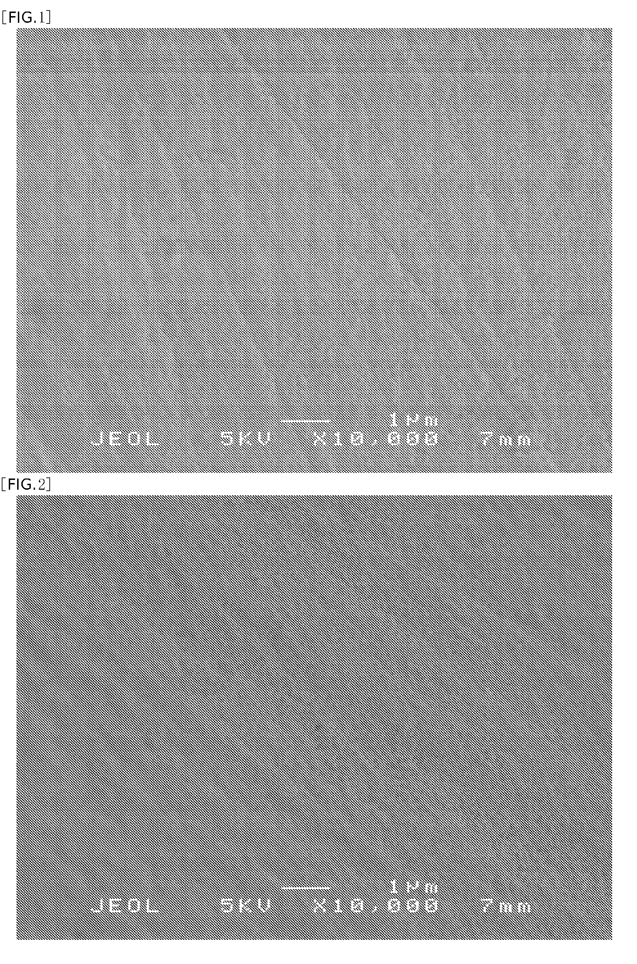

**[0103]** Test conditions: Human teeth were extracted and divided into 1tooth/3 blocks. Then enamel surface of teeth was polished by using #4000 polishing agent. Further, the solvent of 0.5mol EDTA was coated and kept for 60 seconds. Then smear layer was removed and it was considered as the subject's tooth. The tooth was observed under the scanning electron microscope. The drawing of the photograph of the enamel surface observed under the scanning electron microscope is shown in Fig.1. Note that, the scanning electron microscopy has been done after performing gold evaporation. It is flat and smooth as shown in Fig.1.

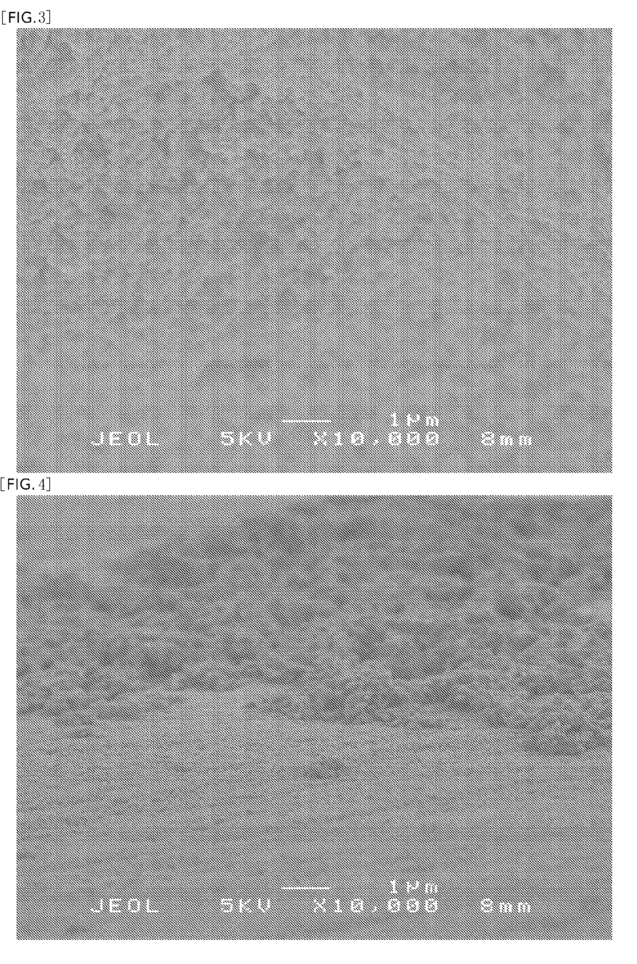

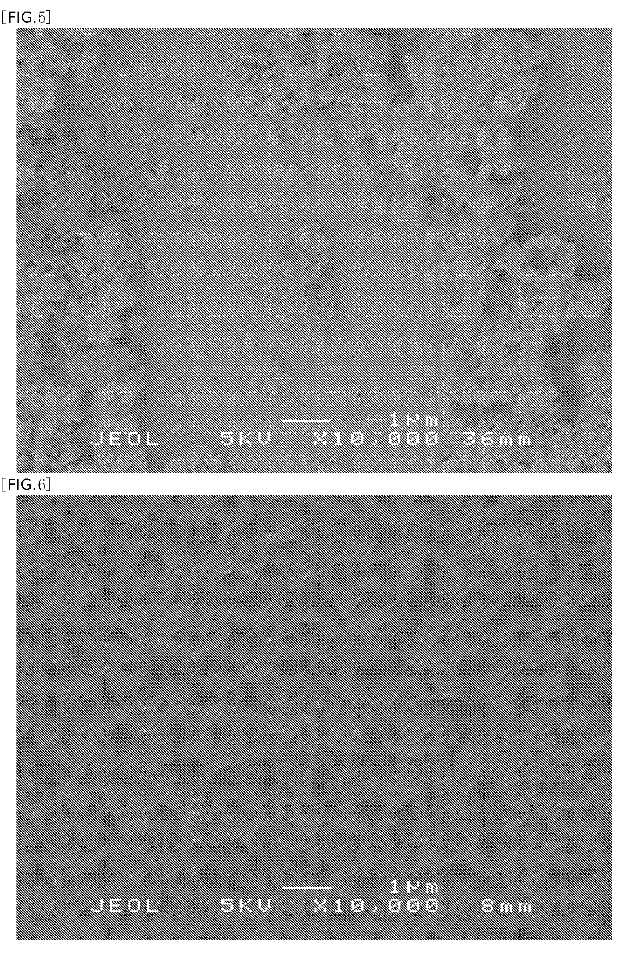

**[0104]** Next, the test drug containing the mixture of each type of fine hydroxyapatite particles 20mg obtained in the manufacturing example 1 to 3 and comparison manufacturing example land purified water 80mg was smeared on the exposed surface of the subject's tooth and naturally dried for 24 hours after coating for 20 seconds. Then it was observed under the scanning electron microscope after performing the gold evaporation. The drawing of the photograph of the enamel surface observed under the scanning electron microscope at that time was shown in Fig.2 to Fig.5. Here, the test result of the fine hydroxyapatite particles are shown wherein, Fig.2 shows that the average particle diameter is 28nm (manufacturing example 1), Fig.3 shows that the average particle diameter is 43nm (manufacturing example 2) and Fig. 4 shows that the average particle diameter is 167nm (manufacturing example 3) Note that, Fig.5 is the test result of unbaked 42nm fine hydroxyapatite particles (comparison manufacturing example 1) When hydroxyapatatie with average diameter 43nm and 28nm were selected as the test drug, it could be confirmed that it is adhered comparatively smoothly. On the other hand, when hydroxyapatatie with average diameter 167nm was selected as the test drug, the flocculated body of the hydroxyapatite was seen and it could be confirmed that the tooth surface on which the particles are adhered is uneven. The flocculation was seen to be large especially in unbaked apatite. Note that, when enamel portion was observed with naked eyes, it was understood that the tooth, on which the fine hydroxyapatite particles with average diameter 28nm and 43nm were applied was white with high luster. The tooth, on which the fine hydroxyapatite particles with average diameter 167nm were applied, was white but the luster was less. There was no luster on tooth on which unbaked particles were applied.

(Adhesion test)

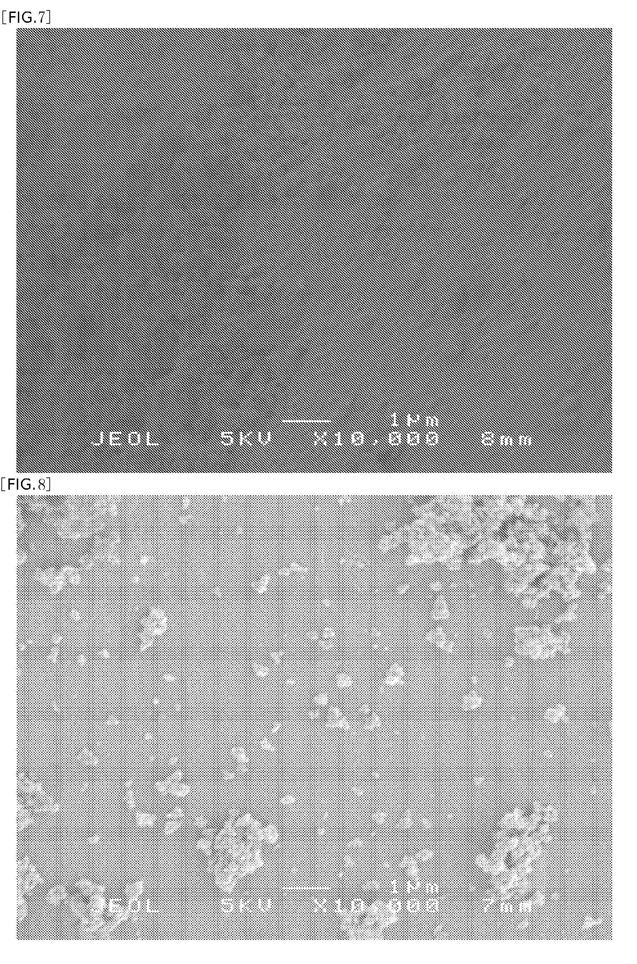

**[0105]** The particles obtained in the manufacturing example 2, 3 and comparison manufacturing example 1 were immersed overnight into the water and adhesion test was performed. Each type of fine hydroxyapatite particles were applied on the enamel surface under the same conditions as that of the coat applying test to enamel and naturally dried for 24 hours. Then the test tooth was immersed into the water and kept overnight. Then, it was naturally dried for further 24 hours and observed under the scanning electron microscope after performing gold evaporation. The drawing of the photograph of the enamel surface observed under the scanning electron microscope at that time is shown in Fig. 6 to Fix.8. Here, Fig.6 shows the test result of the fine hydroxyapatite particles obtained in the manufacturing example 2. Fig. 7 shows the test result of the fine hydroxyapatite particles obtained in the manufacturing example 3 and Fig.8 shows the test results of the fine hydroxyapatite particles obtained in the comparison manufacturing example 1. According to Fig.6 and Fig.7, it was observed that very few fine hydroxyapatite particles have been removed from the surface of the tooth despite keeping overnight into the water. On the other hand, according to Fig.8 it was observed that some portion of the particle has been removed.

(Filler test for small scratches on the tooth surface)

**[0106]** Test conditions: Human teeth were extracted and divided into 1tooth/3 blocks. Then enamel surface of teeth was polished by using #4000 polishing agent. Further, the solvent of 0.5mol EDTA was coated and kept for 60 seconds. Then smear layer was removed and it was considered as the subject's tooth.

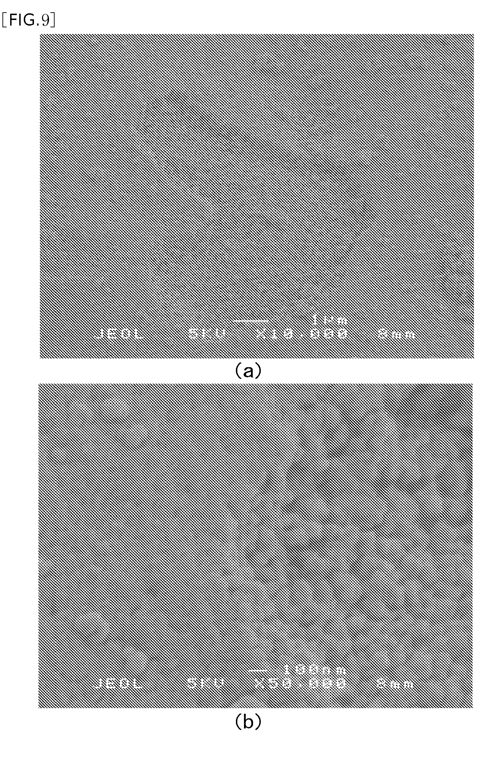

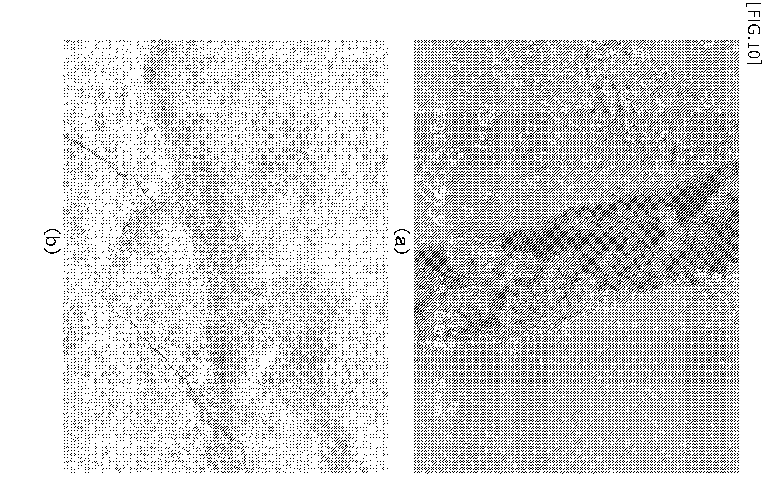

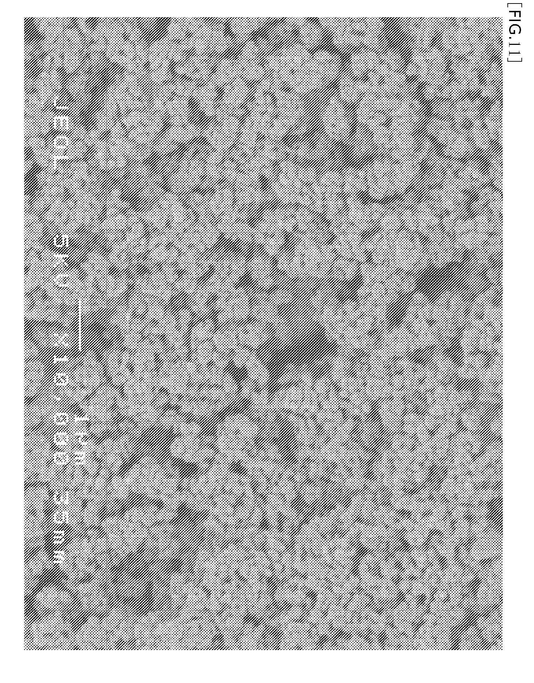

**[0107]** Next, the test drug containing the mixture of each type of fine hydroxyapatite particles 20mg obtained in the manufacturing example 2 and 3 and purified water 80mg was smeared on the exposed surface of the subject's tooth and naturally dried for 24 hours after coating for 20 seconds. Then it was observed under the scanning electron microscope after performing the gold evaporation. The drawing of the photograph of the enamel surface observed under the scanning electron microscope at that time was shown in Fig.9 to Fig.11. Here, the test result of the fine hydroxyapatite particles are shown, wherein Fig.9 and Fig.10 shows that the average particle diameter is 43nm (manufacturing example 2), Fig.

11 shows that the average particle diameter is 167nm (manufacturing example 3). In Fig.9, it was observed that the portions of the beginning tooth decay selectly, wherein 43nm fine hydroxyapatite particles are present on the enamel surface are filled. In addition, as shown in Fig.10, when 43nm fine hydroxyapatite particles were used, it was observed that crack portions of the tooth surface are filled. On the other hand, when fine hydroxyapatite particles with average particle diameter 167nm were selected, the flocculated body of the hydroxyapatite was seen and it was observed that the cracked portion is hardly filled.

(Application test for the dentine)

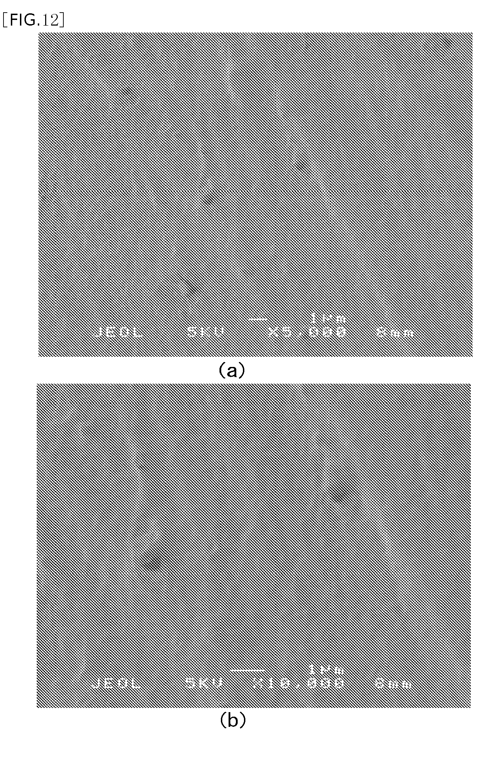

Test conditions: Human teeth were extracted and divided into 1tooth/3 blocks. Then enamel surface was removed and dentine is made to expose. After that #4000 polishing agent was applied on the dentine surface so that it will be smoothly applied on it. Further, the solvent of 0.5mol EDTA was coated and kept for 60 seconds. Then smear layer was removed and it was considered as the subject's tooth. The tooth was observed under the scanning electron microscope. The drawing of the photograph of the dentine surface observed under the scanning electron microscope is shown in Fig.12. Note that, the scanning electron microscopy has been done after performing gold evaporation. As shown in Fig.12, it is understood that the dental tubules have been exposed on the exposure portion. Then, it is understood that the diameter of the dental tubule is $0.9\mu$m.

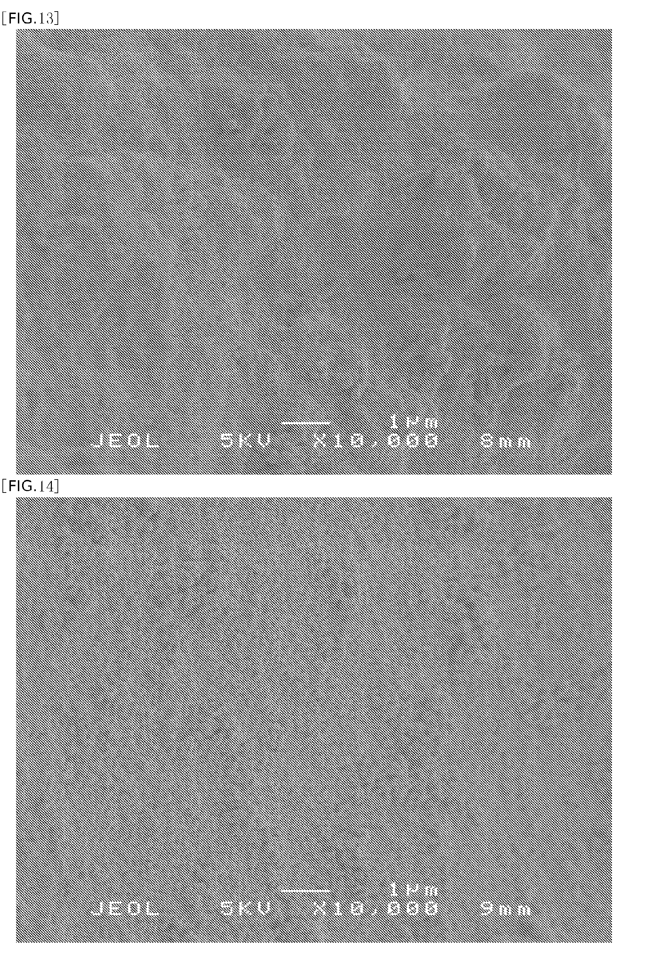

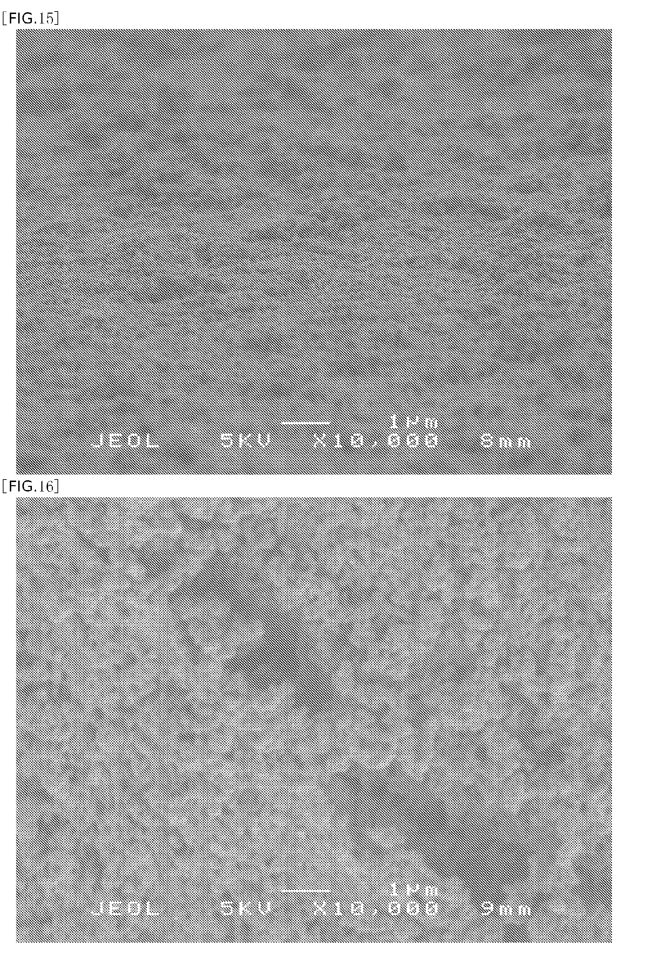

Next, the test drug containing the mixture of each type of fine hydroxyapatite particles 20mg obtained in the manufacturing example 1 to 3 and purified water 80mg was smeared on the exposed surface of the subject's tooth and naturally dried for 24 hours after coating for 20 seconds. Then it was observed under the scanning electron microscope after performing the gold evaporation. The drawing of the photograph of the dentine surface observed under the scanning electron microscope at that time was shown in Fig.13 to Fig.16. Here, the test result of the fine hydroxyapatite particles are shown, wherein Fig.13 shows that the average particle diameter is 28nm (manufacturing example 1), Fig.14 shows that the average particle diameter is 43nm (manufacturing example 2) and Fig.15 shows that the average particle diameter is 167nm (manufacturing example 3). Note that, Fig.16 shows the test result of unbaked 42nm fine hydroxyapatite particles (comparison manufacturing example 1). It could be confirmed that, when hydroxyapatite particles with the average particle diameter 28nm was selected as the test drug, the particles are comparatively smoothly adhered to the tooth surface with some uneven portions. In addition, it could be confirmed that, when hydroxyapatite particles with the average particle diameter 43nm was selected as the test drug, the particles are adhered smoothly and uniformly on the tooth surface. On the other hand, it could be confirmed that, when hydroxyapatite particles with the average particle diameter 167nm was selected as the test drug, the particles are adhered almost uniformly but flocculation of hydroxyapatite particles could be seen at some portions. The tooth surface on which the particles are adhered is somewhat uneven. The flocculation was seen to be large especially in unbaked apatite.

**Claims**

1. A tooth surface repairing material containing fine calcium phosphate particles, wherein the fine calcium phosphate particles are highly crystalline calcium phosphate and have an average particle diameter in the range of 20 to 100 nm.

2. The tooth surface repairing material according to Claim 1, wherein the fine calcium phosphate particles are manufactured by a method which includes a mixing process to mix primary particles containing calcium phosphate and a fusion inhibitor and a sintering process to convert the primary particles included in the particle mixture into highly crystalline calcium phosphate particles by exposing the particle mixture obtained by the mixing process.

3. The tooth surface repairing material according to Claim 2, wherein the fusion inhibitor used in the mixing process contains calcium ions.

4. The tooth surface repairing material according to any of Claims 1 to 3, wherein an abundance ratio (Ca/P) of the calcium atom with respect to the phosphorus atom on the surface of the fine calcium phosphate particles is 1.6 or more.

5. The tooth surface repairing material according to any of Claims 1 to 4, wherein at least some of the fine calcium phosphate particles are in the form of a particle.

6. The tooth surface repairing material according to any of Claims 1 to 5, wherein at least some of the fine calcium phosphate particles are fluroapatite.

7. The tooth surface repairing material according to any one of inventions (1) to (6), wherein at least some of the fine calcium phosphate particles have a coefficient of variation of 20% or less.

[FIG.1]

[FIG.2]

[FIG.3]

[FIG.4]

[FIG.5]

[FIG.6]

[FIG.7]

[FIG.8]

[FIG.9]

(a)

(b)

[FIG.10]

(a)

(b)

[FIG.11]

[FIG.12]

(a)

(b)

[FIG.13]

[FIG.14]

[FIG.15]

[FIG.16]

EP 2 409 677 A1

## INTERNATIONAL SEARCH REPORT

| | International application No. |
|---|---|
| | PCT/JP2009/055478 |

A. CLASSIFICATION OF SUBJECT MATTER
*A61K6/00(2006.01)i*

According to International Patent Classification (IPC) or to both national classification and IPC

B. FIELDS SEARCHED

Minimum documentation searched (classification system followed by classification symbols)
A61K6/00

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho    1971-2009   Toroku Jitsuyo Shinan Koho   1994-2009

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2006/030782 A1 (Japan Science and | 1-5,7 |
| Y | Technology Agency), | 1-7 |
| | 23 March, 2006 (23.03.06), | |
| | Claims 1, 6 to 8, 21; Par. Nos. [0002], [0090], [0175] | |
| | & US 2007/0259181 A1     & EP 1808411 A1 | |
| | & KR 10-2007-0053337 A   & CN 101035741 A | |
| | | |
| Y | JP 09-202717 A (Sangi Co., Ltd.), | 1-7 |
| | 05 August, 1997 (05.08.97), | |
| | Claim 1; Par. No. [0001]; examples 1 to 2 | |
| | & US 5833959 A          & EP 786245 A1 | |
| | & DE 69713175 D | |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 April, 2009 (21.04.09) | 12 May, 2009 (12.05.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

29

**INTERNATIONAL SEARCH REPORT**

International application No.

PCT/JP2009/055478

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-128513 A (President of Osaka University), 09 May, 2002 (09.05.02), Claims 1 to 3; Par. No. [0016] (Family: none) | 1-7 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H04247020 A
- JP H0624929 A
- JP H09202717 A
- JP 2002137910 A
- JP 5001711 A

**Non-patent literature cited in the description**

- *Journal of Dental Health,* 1988, vol. 38, 510-511